# EUROPEAN PATENT SPECIFICATION

(11) **EP 1 707 568 B1**
(45) Date of publication and mention of the grant of the patent: **04.08.2010**
(21) Application number: 05076845.6
(22) Date of filing: 07.02.1992
(51) Int. Cl.: A61K 31/4745, A61P 35/00

(54) **A pharmaceutical composition for use in the treatment of ovarian cancer in a human afflicted therewith**
Pharmazeutische Zusammensetzung zur Verwendung in der Behandlung von Eierstockkrebs
Composition pharmaceutique utilisée dans le traitement du cancer de l'ovaire

(30) Priority: 21.02.1991 US 658948
(43) Date of publication of application: 04.10.2006
(62) Divisional of application: 92907833.5
(73) Proprietor: GlaxoSmithKline LLC, Philadelphia, PA 19102 (US)
(72) Inventor: Johnson, Randall, Keith, c/o GlaxoSmithkline, King of Prussia, Pennsylvania 19406 (US)
(74) Representative: Breen, Anthony Paul

(56) References cited:
- EP-A- 0 321 122
- H. BURRIS ET AL: "SKF 104864: preclinical studies of a new topoisomerase I inhibitor" PROC. ANNU. MEET. AM. ASSOC. CANCER RES., vol. 31, 1990, page 431, XP009096164

## Description

### BACKGROUND OF THE INVENTION

The invention relates to a pharmaceutical composition comprising topotecan, for use in the treatment of ovarian cancer in a human afflicted therewith. This invention also relates to topotecan for use in the treatment of ovarian cancer in a human afflicted therewith by administration to such human of an effective amount of topotecan. Topotecan is a compound of the water soluble camptothecin analog class.

The structure of the DNA helix within eukaryotic cells imposes certain topological problems that the cellular apparatus must solve in order to use its genetic material as a template. The separation of the DNA strands is fundamental to cellular processes such as DNA replication and transcription. Since eukaryotic DNA is organized into chromatin by chromosomal proteins, the ends are constrained and the strands cannot unwind without the aid of enzymes that alter topology. It has long been recognized that the advancement of the transcription or replication complex along the DNA helix would be facilitated by a swivel point which would relieve the torsional strain generated during these processes.

Topoisomerases are enzymes that are capable of altering DNA topology in eukaryotic cells. They are critical for important cellular functions and cell proliferation. There are two classes of topoisomerases in eukaryotic cells, type I and type II.

Topoisomerase I is a monomeric enzyme of approximately 100,000 molecular weight. The enzyme binds to DNA and introduces a transient single-strand break, unwinds the double helix (or allows it to unwind), and subsequently reseals the break before dissociating from the DNA strand.

Camptothecin, a water-insoluble alkaloid produced by trees indigenous to China and India, and a few other congeners thereof, are the only class of compounds known to inhibit topoisomerase I.

Camptothecin and other topoisomerase I inhibiting congeners have not proven to be attractive for clinical drug development as cytolytic agents because of lack of clinical efficacy, unacceptable dose-limiting toxicity, unpredictable toxicity, poor aqueous solubility, and/or unacceptable shelf life stability.

Therefore, there is a need for topoisomerase I inhibiting agents which avoid the aforementioned undesirable features of camptothecin and related topoisomerase I inhibiting congeners. Topotecan, a compound of the water soluble camptothecin analog class, is a specific inhibitor of DNA topoisomerase I which fulfills such need.

U.S. Patent Number 5,004,758 and EP 0 321 122 A1 disclose a series of compounds which are water soluble camptothecin analogs. H. Burris et al., Proc. Am. Assoc. Cancer Res., March 1990, vol.31, p.431, abstract 2558 discloses the results of preclinical studies of SKF 104864, a semisynthetic analog of the natural product camptothecin which exerts its antineoplastic activity through inhibition of topoisomerase I, in a human tumor clonogenic assay.

### SUMMARY OF THE INVENTION

This invention relates to topotecan for use in the treatment of ovarian cancer in a human afflicted therewith by intravenous administration to such human employing a course of therapy of from 1.0 to 2 mg of topotecan/m² of body surface area per day for five consecutive days,
wherein the term "ovarian cancer" means adenocarcinoma of the ovary.

The invention also relates to a pharmaceutical composition comprising topotecan, for use in the treatment of ovarian cancer in a human afflicted therewith, wherein the composition is for intravenous administration to such human employing a course of therapy of from 1.0 to 2 mg of topotecan/m² of body surface area per day for five consecutive days,
wherein the term "ovarian cancer" means adenocarcinoma of the ovary.

### DETAILED DESCRIPTION OF THE INVENTION

By the term "a compound of the water soluble camptothecin analog class" is meant a compound of the formula: 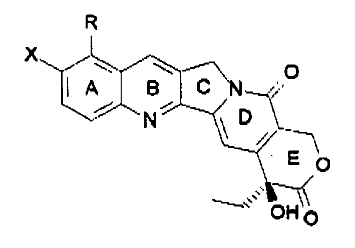 wherein:
a) X is hydroxy and R is trimethylammoniummethyl;
b) X is hydroxy and R is N-methylpiperazinylmethyl;
c) X is hydroxy and R is N-methylanilinomethyl;
d) X is hydroxy and R is cyclohexylaminomethyl;
e) X is hydroxy and R is N,N-dimethylaminoethyloxymethyl;
f) X is hydroxy and R is cyclopropylaminomethyl;
g) X is hydroxy and R is morpholinomethyl;
h) X is hydroxy and R is aminomethyl;
i) X is hydroxy and R is cyanomethyl; or
j) X is hydroxy and R is dimethylaminomethyl; or any pharmaceutically acceptable salts, hydrates and solvates thereof.

The preparation of any compound of the water soluble camptothecin analog class (including pharmaceutically acceptable salts, hydrates and solvates thereof) as well as the preparation of oral and parenteral pharmaceutical compositions comprising a compound of the water soluble camptothecin analog class and an inert, pharmaceutically acceptable carrier or diluent, is extensively described in U.S. Patent Number 5,004,758. The same extensive description is found in European Patent Application Number 88311366.4, published on June 21, 1989 as Publication Number EP 0 321 122.

Topotecan is the compound of the water soluble camptothecin analog class which is used in the present invention.

By the term "topotecan" as used herein is meant the compound of the formula:

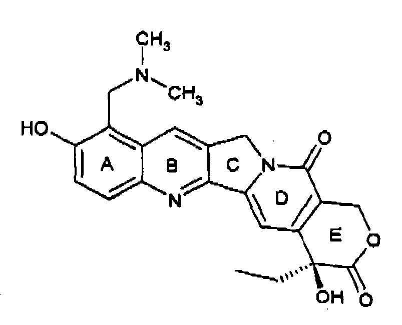

(S)-9-dimethylaminomethyl-10-hydroxycamptothecin or any pharmaceutically acceptable salt, hydrate or solvate thereof. Topotecan's chemical name is (S)-10[(dimethylamino)methyl]-9-ethyl-4,9-dihydroxy-1H-pyrano[3',4':6,7]indolizino[1,2-b]quinolone-3,14(4H,12H)-dione.

Topotecan is water-soluble by virtue of the presence of the basic side-chain at position 9 which forms salts with acids. Preferred salt forms of topotecan include the hydrochloride salt, acetate salt and methanesulfonic acid salt. An alkali metal salt form of the carboxylate formed on alkaline hydrolysis of the E-ring lactone of topotecan would also yield a soluble salt, such as the sodium salt.

The preparation of topotecan (including pharmaceutically acceptable salts, hydrates and solvates thereof) as well as the preparation of oral and parenteral pharmaceutical compositions comprising topotecan and an inert, pharmaceutically acceptable carrier or diluent, is extensively described in U.S. Patent Number 5,004,758. The same extensive description is found in European Patent Application Number 88311366.4, published on June 21, 1989 as Publication Number EP 0 321 122.

The invention can be used in a method of treating ovarian cancer in a human afflicted therewith which comprises administering to such human an effective amount of topotecan.

By the term "ovarian cancer" as used herein is meant adenocarcinoma of the ovary.

By the term "treating ovarian cancer" as used herein is meant the inhibition of the growth of ovarian cancer cells. Preferably such treatment also leads to the regression of tumor growth, i.e., the decrease in size of a measurable tumor. Most preferably, such treatment leads to the complete regression of the tumor.

By the term "administering" is meant parenteral or oral administration. By "parenteral" is meant intravenous, subcutaneous and intramuscular administration.

By the term "effective amount of topotecan" as used herein is meant a course of therapy which will result in treating ovarian cancer. It will be appreciated that the actual preferred course of therapy will vary according to, inter alia, the mode of administration, the particular formulation of topotecan being utilized, the mode of administration and the particular host being treated. The optimal course of therapy for a given set of conditions can be ascertained by those skilled in the art using conventional course of therapy determination tests in view of the information set out herein, as well as the information outlined in U.S. Patent Number 5,004,758. The same information is found in European Patent Application Number 88311366.4, published on June 21, 1989 as Publication Number EP 0 321 122.

For parenteral administration of a compound of the water soluble camptothecin analog class being topotecan, the course of therapy employed is from 1.0 to 2 mg/m² of body surface area per day for five consecutive days. Most preferably, the course of therapy employed is from about 1.5 to 2 mg/m² of body surface area per day for five consecutive days. Preferably, the course of therapy is repeated at least once at about a seven day to about a twenty-eight day interval (from the date of initiation of therapy) depending upon the initial dosing schedule and the patient's recovery of normal tissues. Most preferably, the course of therapy continues to be repeated based on tumor response.

Preferably, the parenteral administration will be by short (e.g., 30 minute) or prolonged (e.g., 24 hour) intravenous infusion. More preferably, the topotecan will be administered by a 30 minute intravenous infusion.

At this time, it is believed that the most preferred course of parenteral therapy to be employed with topotecan for a previously non-treated or lightly pretreated patient is an initial course of therapy of 1.5 mg of topotecan/m² of body surface area per day administered by short intravenous infusion for five consecutive days. When the patient has recovered sufficiently from the drug-related effects of this initial course, an additional course of therapy of 2.0 mg of topotecan/m² of body surface area per day is administered by short intravenous infusion for five consecutive days, to be repeated based on tumor response.

At this time, it is believed that the most preferred course of parenteral therapy to be employed with topotecan for a heavily pretreated patient is an initial course of therapy of 1.0 mg of topotecan/m² of body surface area per day administered by short intravenous infusion for five consecutive days. When the patient has recovered sufficiently from the drug-related effects of this initial course, an additional course of therapy of 1.5 mg of topotecan/m² of body surface area per day is administered by short intravenous infusion for five consecutive days, such course of therapy to be repeated based on tumor response.

### Clinical Pharmaceutical Information

Topotecan is currently undergoing Phase I clinical investigation. The following pharmaceutical information is being supplied to the clinicians:
How supplied - As a vial containing 5 mg (of the base) with 100 mg mannitol. The pH is adjusted to 3.0 with HCl/NaOH. Lyophilized powder is light yellow in color. Intact vials should be stored under refrigeration (2-8 degrees Centigrade).
Solution Preparation -When the 5 mg vial is reconstituted with 2 ml of Sterile Water for Injection, USP, each ml will contain 2.5 mg of topotecan as the base and 50 mg of mannitol, USP. Topotecan must not be diluted or mixed with buffered solutions because of solubility and stability considerations.
Stability - Shelf life surveillance of the intact vials is ongoing. Because the single-use lyophilized dosage form contains no antibacterial preservatives, it is advised that the reconstituted solution be discarded eight hours after initial entry into the vial. Further dilutions of the reconstituted solution to concentrations of 0.02 mg/ml and 0.1 mg/ml in 5% Dextrose Injection, USP, ("D5W") or 0.9% Sodium Chloride Injection, USP, ("NS") in plastic bags stored at room temperature yielded the following stability results:

**Percentage of Initial Topotecan Remaining in Solution**

| Diluent | Time (hrs) | Concentration | |
|---|---|---|---|
| | | 0.02 mg/ml | 0.1 mg/ml |
| D5W | 0 | 100.00 | 100.00 |
| | 6 | 99.29 | 99.68 |
| | 24 | 102.30 | 98.16 |
| | 48 | 101.98 | 97.91 |
| | | | |
| NS | 0 | 100.00 | 100.00 |
| | 6 | 98.58 | 97.71 |
| | 24 | 96.01 | 98.30 |
| | 48 | 102.03 | 98.35 |

Topotecan diluted in saline (10 ug/ml or 500 ug/ml) or dextrose (6.7 ug/ml or 330 ug/ml) is stable in a hang-bag for 24 hours with at least 95% recovery.
Treatment dose - The treatment dose is to be diluted in a final volume of 150 ml of Sodium Chloride Injection, USP (without preservatives) and administered over a 30 minute period. The treatment dose is to be kept under refrigeration and protected from light and it is to be used within 24 hours.

### Utility

One human patient with ovarian cancer, who was refractory to two previous platinum-containing regimens (i.e., cisplatin-cyclophosphamide combination regimen and treatment with single agent carboplatin), received a course of therapy comprising intravenous administration of 1.5 mg of topotecan/m² of body surface area per day for five consecutive days. This course of therapy was repeated weekly nine more times to date at twenty-one day intervals (from the date of initiation of therapy) for a total of ten treatments. Tumor size regression was evaluated by CAT (computerized axial tomography) scan. Tumor size regression was observed following two courses of therapy of the above-outlined treatment regimen. An even greater response was observed following four courses. At least until the tenth treatment, this clinically significant response was sustained, i.e., a greater than fifty percent (50%) tumor size regression was obtained.

## Claims

1. A pharmaceutical composition comprising topotecan, for use in the treatment of ovarian cancer in a human afflicted therewith, wherein the composition is for intravenous administration to such human employing a course of therapy of from 1.0 to 2 mg of topotecan/m² of body surface area per day for five consecutive days,
wherein the term "ovarian cancer" means adenocarcinoma of the ovary.

2. A pharmaceutical composition for use in the treatment of ovarian cancer as claimed in claim 1, wherein the topotecan is in the salt form of the hydrochloride salt, acetate salt or methanesulfonic acid salt.

3. A pharmaceutical composition for use in the treatment of ovarian cancer as claimed in claim 1 or 2, which is a parenteral pharmaceutical composition comprising topotecan and an inert, pharmaceutically acceptable carrier or diluent.

4. A pharmaceutical composition for use in the treatment of ovarian cancer as claimed in Claim 1, 2 or 3, which is for intravenous administration to such human employing a course of therapy of from 1.5 to 2 mg of topotecan/m² of body surface area per day for five consecutive days.

5. A pharmaceutical composition for use in the treatment of ovarian cancer as claimed in Claim 4, which is for intravenous administration to such human employing a course of therapy of 1.5 mg of topotecan/m² of body surface area per day for five consecutive days.

6. A pharmaceutical composition for use in the treatment of ovarian cancer as claimed in any one of Claims 1 to 5, which is for repetition of the course of therapy at least once at a seven day to a twenty-eight day interval (from the date of initiation of therapy).

7. A pharmaceutical composition for use in the treatment of ovarian cancer as claimed in any one of Claims 1 to 6, wherein the intravenous administration is by short or prolonged intravenous infusion.

8. A pharmaceutical composition for use in the treatment of ovarian cancer as claimed in Claim 7, wherein the intravenous administration is by short intravenous infusion.

9. A pharmaceutical composition for use in the treatment of ovarian cancer as claimed in Claim 8, wherein the short intravenous infusion is a 30 minute intravenous infusion.

10. A pharmaceutical composition for use in the treatment of ovarian cancer as claimed in Claim 7, which is for intravenous administration by prolonged intravenous infusion, and wherein the prolonged intravenous infusion is a 24 hour intravenous infusion.

11. Topotecan for use in the treatment of ovarian cancer in a human afflicted therewith by intravenous administration to such human employing a course of therapy of from 1.0 to 2 mg of topotecan/m² of body surface area per day for five consecutive days
wherein the term "ovarian cancer" means adenocarcinoma of the ovary.

12. Topotecan for use in the treatment of ovarian cancer as claimed in claim 11, wherein the topotecan is in the salt form of the hydrochloride salt, acetate salt or methanesulfonic acid salt.

13. Topotecan for use in the treatment of ovarian cancer as claimed in Claim 11 or 12, which is for intravenous administration to such human employing a course of therapy of from 1.5 to 2 mg of topotecan/m² of body surface area per day for five consecutive days.

14. Topotecan for use in the treatment of ovarian cancer as claimed in Claim 13, which is for intravenous administration to such human employing a course of therapy of 1.5 mg of topotecan/m² of body surface area per day for five consecutive days.

15. Topotecan for use in the treatment of ovarian cancer as claimed in any one of Claims 11 to 14, which is for repetition of the course of therapy at least once at a seven day to a twenty-eight day interval (from the date of initiation of therapy).

16. Topotecan for use in the treatment of ovarian cancer as claimed in any one of Claims 11 to 15, wherein the intravenous administration is by short or prolonged intravenous infusion.

17. Topotecan for use in the treatment of ovarian cancer as claimed in Claim 16, wherein the intravenous administration is by short intravenous infusion.

18. Topotecan for use in the treatment of ovarian cancer as claimed in Claim 17, wherein the short intravenous infusion is a 30 minute intravenous infusion.

19. Topotecan for use in the treatment of ovarian cancer as claimed in Claim 16, wherein the intravenous administration is by prolonged intravenous infusion, wherein the prolonged intravenous infusion is a 24 hour intravenous infusion.

## Patentansprüche

1. Ein Arzneimittel, umfassend Topotecan, zur Verwendung in der Behandlung von Ovarialkrebs in einem davon betroffenen Menschen, wobei die Zusammensetzung für die intravenöse Verabreichung an einen solchen Menschen vorgesehen ist, wobei ein Therapieverlauf angewendet wird, welcher 1,0 bis 2 mg Topotecan/m² Körperoberfläche pro Tag an fünf aufeinanderfolgenden Tagen umfasst,
wobei der Begriff "Ovarialkrebs" Adenocarcinom des Ovars bedeutet.

2. Ein Arzneimittel zur Verwendung in der Behandlung von Ovarialkrebs wie in Anspruch 1 beansprucht, wobei das Topotecan in der Salzform des Hydrochloridsalzes, Acetatsalzes oder Methansulfonsäuresalzes vorliegt.

3. Ein Arzneimittel zur Verwendung in der Behandlung von Ovarialkrebs wie in Anspruch 1 oder 2 beansprucht, welches ein parenterales Arzneimittel, umfassend Topotecan und ein(en) inertes(n), pharmazeutisch verträgliches(n) Träger oder Verdünnungsmittel, ist.

4. Ein Arzneimittel zur Verwendung in der Behandlung von Ovarialkrebs wie in Anspruch 1, 2 oder 3 beansprucht, welches für die intravenöse Verabreichung an einen solchen Menschen vorgesehen ist, wobei ein Therapieverlauf angewendet wird, welcher 1,5 bis 2 mg Topotecan/m² Körperoberfläche pro Tag an fünf aufeinanderfolgenden Tagen umfasst.

5. Ein Arzneimittel zur Verwendung in der Behandlung von Ovarialkrebs wie in Anspruch 4 beansprucht, welches für die intravenöse Verabreichung an einen solchen Menschen vorgesehen ist, wobei ein Therapieverlauf angewendet wird, welcher 1,5 mg Topotecan/m² Körperoberfläche pro Tag an fünf aufeinanderfolgenden Tagen umfasst.

6. Ein Arzneimittel zur Verwendung in der Behandlung von Ovarialkrebs wie in einem der Ansprüche 1 bis 5 beansprucht, welches für die mindestens einmalige Wiederholung des Therapieverlaufs in einem 7-Tage- bis 28-Tage-Intervall (ab dem Tag des Therapiebeginns) vorgesehen ist.

7. Ein Arzneimittel zur Verwendung in der Behandlung von Ovarialkrebs wie in einem der Ansprüche 1 bis 6 beansprucht, wobei die intravenöse Verabreichung durch kurze oder lange intravenöse Infusion erfolgt.

8. Ein Arzneimittel zur Verwendung in der Behandlung von Ovarialkrebs wie in Anspruch 7 beansprucht, wobei die intravenöse Verabreichung durch kurze intravenöse Infusion erfolgt.

9. Ein Arzneimittel zur Verwendung in der Behandlung von Ovarialkrebs wie in Anspruch 8 beansprucht, wobei die kurze intravenöse Infusion eine intravenöse 30-Minuten-Infusion ist.

10. Ein Arzneimittel zur Verwendung in der Behandlung von Ovarialkrebs wie in Anspruch 7 beansprucht, welches zur intravenösen Verabreichung durch lange intravenöse Infusion vorgesehen ist und wobei die lange intravenöse Infusion eine intravenöse 24-Stunden-Infusion ist.

11. Topotecan zur Verwendung in der Behandlung von Ovarialkrebs in einem davon betroffenen Menschen durch intravenöse Verabreichung an einen solchen Menschen, wobei ein Therapieverlauf angewendet wird, welcher 1,0 bis 2 mg Topotecan/m² Körperoberfläche pro Tag an fünf aufeinanderfolgenden Tagen umfasst,
wobei der Begriff "Ovarialkrebs" Adenocarcinom des Ovars bedeutet.

12. Topotecan zur Verwendung in der Behandlung von Ovarialkrebs wie in Anspruch 11 beansprucht, wobei das Topotecan in der Salzform des Hydrochloridsalzes, Acetatsalzes oder Methansulfonsäuresalzes vorliegt.

13. Topotecan zur Verwendung in der Behandlung von Ovarialkrebs wie in Anspruch 11 oder 12 beansprucht, welches für die intravenöse Verabreichung an einen solchen Menschen vorgesehen ist, wobei ein Therapieverlauf angewendet wird, welcher 1,5 bis 2 mg Topotecan/m² Körperoberfläche pro Tag an fünf aufeinanderfolgenden Tagen umfasst.

14. Topotecan zur Verwendung in der Behandlung von Ovarialkrebs wie in Anspruch 13 beansprucht, welches für die intravenöse Verabreichung an einen solchen Menschen vorgesehen ist, wobei ein Therapieverlauf angewendet wird, welcher 1,5 mg Topotecan/m² Körperoberfläche pro Tag an fünf aufeinanderfolgenden Tagen umfasst.

15. Topotecan zur Verwendung in der Behandlung von Ovarialkrebs wie in einem der Ansprüche 11 bis 14 beansprucht, welches für die mindestens einmalige Wiederholung des Therapieverlaufs in einem 7-Tage- bis 28-Tage-Intervall (ab dem Tag des Therapiebeginns) vorgesehen ist.

16. Topotecan zur Verwendung in der Behandlung von Ovarialkrebs wie in einem der Ansprüche 11 bis 15 beansprucht, wobei die intravenöse Verabreichung durch kurze oder lange intravenöse Infusion erfolgt.

17. Topotecan zur Verwendung in der Behandlung von Ovarialkrebs wie in Anspruch 16 beansprucht, wobei die intravenöse Verabreichung durch kurze intravenöse Infusion erfolgt.

18. Topotecan zur Verwendung in der Behandlung von Ovarialkrebs wie in Anspruch 17 beansprucht, wobei die kurze intravenöse Infusion eine intravenöse 30-Minuten-Infusion ist.

19. Topotecan zur Verwendung in der Behandlung von Ovarialkrebs wie in Anspruch 16 beansprucht, wobei die intravenöse Verabreichung durch lange Infusion vorgesehen ist, wobei die lange intravenöse Infusion eine intravenöse 24-Stunden-Infusion ist.

## Revendications

1. Composition pharmaceutique comprenant du topotécan, destinée à être utilisée dans le traitement du cancer des ovaires chez un être humain qui en est atteint, ladite composition étant destinée à l'administration intraveineuse à cet être humain en utilisant un schéma thérapeutique de 1,0 à 2 mg de topotécan/m² de surface corporelle par jour pendant cinq jours consécutifs,
l'expression "cancer des ovaires" désignant l'adénocarcinome de l'ovaire.

2. Composition pharmaceutique destinée à être utilisée dans le traitement du cancer des ovaires suivant la revendication 1, dans laquelle le topotécan est sous forme d'un sel consistant en le chlorhydrate, l'acétate ou le méthanesulfonate.

3. Composition pharmaceutique destinée à être utilisée dans le traitement du cancer des ovaires suivant la revendication 1 ou 2, qui est une composition pharmaceutique parentérale comprenant du topotécan et un support ou diluant inerte pharmaceutiquement acceptable.

4. Composition pharmaceutique destinée à être utilisée dans le traitement du cancer des ovaires suivant la revendication 1, 2 ou 3, qui est destinée à l'administration intraveineuse à un tel être humain en utilisant un schéma thérapeutique de 1,5 à 2 mg de topotécan/m² de surface corporelle par jour pendant cinq jours consécutifs.

5. Composition pharmaceutique destinée à être utilisée dans le traitement du cancer des ovaires suivant la revendication 4, qui est destinée à l'administration intraveineuse à un tel être humain en utilisant un schéma thérapeutique de 1,5 mg de topotécan/m² de surface corporelle par jour pendant cinq jours consécutifs.

6. Composition pharmaceutique destinée à être utilisée dans le traitement du cancer des ovaires suivant l'une quelconque des revendications 1 à 5, qui est destinée à la répétition du schéma thérapeutique au moins une fois à un intervalle de sept jours à vingt-huit jours (depuis la date de début de la thérapie).

7. Composition pharmaceutique destinée à être utilisée dans le traitement du cancer des ovaires suivant l'une quelconque des revendications 1 à 6, dans laquelle l'administration intraveineuse est effectuée par une perfusion intraveineuse brève ou prolongée.

8. Composition pharmaceutique destinée à être utilisée dans le traitement du cancer des ovaires suivant la revendication 7, dans laquelle l'administration intraveineuse est effectuée par brève perfusion intraveineuse.

9. Composition pharmaceutique destinée à être utilisée dans le traitement du cancer des ovaires suivant la revendication 8, dans laquelle la brève perfusion intraveineuse est une perfusion intraveineuse de 30 minutes.

10. Composition pharmaceutique destinée à être utilisée dans le traitement du cancer des ovaires suivant la revendication 7, qui est destinée à l'administration intraveineuse par perfusion intraveineuse prolongée, ladite perfusion intraveineuse prolongée étant une perfusion intraveineuse de 24 heures.

11. Topotécan destiné à être utilisé dans le traitement du cancer des ovaires chez un être humain qui en est atteint, par administration intraveineuse à cet être humain, en utilisant un schéma thérapeutique de 1,0 à 2 mg de topotécan/m² de surface corporelle par jour pendant cinq jours consécutifs,
l'expression "cancer des ovaires" désignant l'adénocarcinome de l'ovaire.

12. Topotécan destiné à être utilisé dans le traitement du cancer des ovaires suivant la revendication 11, ledit topotécan étant sous forme d'un sel consistant en le chlorhydrate, l'acétate ou le méthanesulfonate.

13. Topotécan destiné à être utilisé dans le traitement du cancer des ovaires suivant la revendication 11 ou 12, qui est destiné à l'administration intraveineuse à un tel être humain en utilisant un schéma thérapeutique de 1,5 à 2 mg de topotécan/m² de surface corporelle par jour pendant cinq jours consécutifs.

14. Topotécan destiné à être utilisé dans le traitement du cancer des ovaires suivant la revendication 13, qui est destiné à l'administration intraveineuse à un tel être humain en utilisant un schéma thérapeutique de 1,5 mg de topotécan/m² de surface corporelle par jour pendant cinq jours consécutifs.

15. Topotécan destiné à être utilisé dans le traitement du cancer des ovaires suivant l'une quelconque des revendications 11 à 14, qui est destiné à la répétition du schéma thérapeutique au moins une fois à un intervalle de sept jours à vingt-huit jours (depuis la date de début de la thérapie).

16. Topotécan destiné à être utilisé dans le traitement du cancer des ovaires suivant l'une quelconque des revendications 11 à 15, l'administration intraveineuse étant effectuée par perfusion intraveineuse brève ou prolongée.

17. Topotécan destiné à être utilisé dans le traitement du cancer des ovaires suivant la revendication 16, l'administration intraveineuse étant effectuée par brève perfusion intraveineuse.

18. Topotécan destiné à être utilisé dans le traitement du cancer des ovaires suivant la revendication 17, la brève perfusion intraveineuse étant une perfusion intraveineuse de 30 minutes.

19. Topotécan destiné à être utilisé dans le traitement du cancer des ovaires suivant la revendication 16, l'administration intraveineuse étant effectuée par perfusion intraveineuse prolongée, la perfusion intraveineuse prolongée étant une perfusion intraveineuse de 24 heures.
